# EUROPÄISCHE PATENTSCHRIFT

(11) **EP 1 061 891 B1**
(45) Veröffentlichungstag und Bekanntmachung des Hinweises auf die Patenterteilung: **11.08.2004**
(21) Anmeldenummer: 99910329.4
(22) Anmeldetag: 04.03.1999
(51) Int. Cl.: A61K 7/09

(54) **VERFAHREN ZUM DAUERWELLEN UND FÄRBEN DER HAARE**
METHOD FOR PERMING AND DYEING HAIR
PROCEDE POUR REALISER UNE PERMANENTE ET COLORER LES CHEVEUX

(30) Priorität: 13.03.1998 DE 19810887
(43) Veröffentlichungstag der Anmeldung: 27.12.2000
(73) Patentinhaber: Henkel Kommanditgesellschaft auf Aktien, 40589 Düsseldorf (DE)
(72) Erfinder: HÖFFKES, Horst, D-40595 Düsseldorf (DE); BÜTTNER, Roswitha, D-40477 Düsseldorf (DE); MÖLLER, Hinrich, D-40789 Monheim (DE); OBERKOBUSCH, Doris, D-40591 Düsseldorf (DE)
(86) Internationale Anmeldenummer: PCT/EP1999/001410
(87) Internationale Veröffentlichungsnummer: WO 1999/047107

(56) Entgegenhaltungen:
- EP-A- 0 359 465
- GB-A- 773 559
- US-A- 5 094 662

## Beschreibung

Die Erfindung betrifft ein Verfahren zur gleichzeitigen dauerhaften Verformung und Färbung der Haare, bei dem die Zubereitung zur keratinreduzierenden Stufe eine keratinreduzierende Substanz und eine reaktive Carbonylverbindung sowie gegebenenfalls zusätzlich eine Verbindung aus der Gruppe der aliphatischen oder aromatischen Amine, der Phenole, Aminophenole oder der stickstoffhaltigen Heterocyclen enthält.

Die dauerhafte Haarverformung wird nach den bekannten Dauerwellverfahren in der Weise durchgeführt, daß man das Haar mechanisch verformt und die Verformung z.B. durch Aufwickeln auf Haarwickler oder Papilloten festlegt. Vor oder nach dieser Verformung behandelt man das Haar mit der wäßrigen Zubereitung einer keratinreduzierenden Substanz, spült nach einer Einwirkungszeit mit Wasser und behandelt dann in einem zweiten Schritt mit der wäßrigen Zubereitung eines Oxidationsmittels (Fixierlösung) und spült auch dieses nach einer Einwirkungszeit wieder aus dem Haar aus. Nach dem Trocknen befreit man das Haar wieder von den mechanischen Verformungshilfen (Wickler, Papilloten).

Aus der Literatur sind eine Reihe von Verfahren bekannt, nach denen das Haar in einer Sitzung dauergewellt und gefärbt werden kann. Dabei wird z.B. gemäß EP 260 716 A1 der Fixierlösung ein Oxidationshaarfärbemittel zur gleichzeitigen Färbung der dauergewellten Haare zugesetzt. In anderen Verfahren wird Dauerwellung und Färbung wie üblich aber nacheinander durchgeführt, was die Prozedur sehr aufwendig und zeitraubend macht. Ein Nachteil der gleichzeitigen oxidativen Fixierung und Oxidationsfärbung besteht darin, daß zur Fixierung bevorzugt im schwach sauren Medium (pH = 3 - 5) gearbeitet werden soll, während viele Oxidationsfarbstoffe im alkalischen Medium (pH = 8 - 10) stärker in das Haar eindringen und intensiver färben.

Dies gilt z.B. für so bekannte Entwickler wie p-Aminophenol oder 1,2,4,5-Tetraaminopyrimidin, so daß insbesondere im Orange- und Rot-Bereich für die gleichzeitige Dauerwellung und Färbung ein Bedarf an einer Erweiterung des Nuancenspektrums besteht.

Es wurde nun gefunden. daß sich Haarfarbstoff-Vorprodukte vom Typ der reaktiven Carbonylverbindungen besonders gut dazu eignen, schon mit der keratinreduzierenden Zubereitung der üblichen Haarverformungsverfahren auf das Haar aufgebracht zu werden und dort bevorzugt gelbe, rötliche, braune und blaue Nuancen zu erzeugen.

Gegenstand der Erfindung ist daher ein Verfahren zur gleichzeitigen dauerhaften Verformung und Färbung der Haare, bei dem die Haare vor und/oder nach einer mechanischen Verformung mit einem Keratinreduktionsmittel behandelt, dann gespült und mit einem Oxidationsmittel fixiert werden, wobei man zur Keratinreduktion eine wäßrige Zubereitung verwendet, die eine Mercaptoverbindung oder ein Sulfit und wenigstens einen Farbstoff vom Typ der reaktiven Carbonylverbindungen, ausgewählt aus der Gruppe der aromatischen, heteroaromatischen oder ungesättigten Aldehyde oder Ketone, der Dialdehyde oder Diketone oder der Acetale, Halbaminale oder Iminderivate solcher reaktiver Carbonylverbindungen enthält.

Haarfarbstoffe vom Typ der reaktiven Carbonylverbindungen sind seit längerem bekannt. Geeignete Verbindungen vom Typ der aromatischen Aldehyde sind z.B. in der deutschen Patentanmeldung DE 19 630 274 A1 und DE 19 630 275 A1 beschrieben. Geeignete Verbindungen sind z.B. der 2-Hydroxybenzaldehyd, der 4-Hydroxy-3-methoxy-benzaldehyd (Vanillin) und der 4-Hydroxy-3-methoxy-cinnamaldehyd (Coniferylaldehyd).

Geeignete Verbindungen vom Typ der heteroaromatischen Aldehyde sind z.B. in der deutschen Patentanmeldung DE 19 717 280.6 beschrieben. Besonders gut geeignete Farbstoffe für das erfindungsgemäße Verfahren sind z.B. trans-ß-(2-Furyl)-acrolein, 1-Methylindol-3-aldehyd, 2-(1,3,3-Trimethyl-2-indolinyliden)-acetaldehyd oder Antipyrin-4-aldehyd. Spezielle Produkte dieses Typs mit einer Pyridinium-Gruppe sind in der deutschen Patentanmeldung DE 19 745 356.2 beschrieben, z.B. die sehr gut geeigneten 4-Formyl-1-methylpyridinium-benzolsulfonat und 4-Formyl-1-methylchinolinium-methansulfonat bzw. -methylsulfat.

Geeignete Farbstoffe vom Typ der ungesättigten Aldehyde sind z.B. in der deutschen Patentanmeldung DE 19 717 224.5 beschrieben. Für die vorliegende Erfindung eignet sich besonders gut der Glutaconaldehyd in Form seiner Salze, z.B. seines Alkali- oder Tetrabutylammoniumsalzes oder der 2-Chlor-3-hydroxymethylen-1-cyclohexen-1-aldehyd.

Dialdehyde und Diketone und deren Derivate, die sich als Farbstoffe für das erfindungsgemäße Verfahren eignen, sind z.B. alicyclische und cyclische 1,2- und 1,3-Dicarbonylverbindungen, wie Isatin, Ninhydrin, Alloxan, Isobarbitursäure, p- und o-Chinone, 1,3-Indandione und deren Derivate. Solche Farbstoffe finden sich z.B. in der deutschen Offenlegungsschrift DE 43 35 627 A1. Geeignete Verbindungen sind z.B. der Malondialdehyd, bevorzugt in Form seines Dimethylacetals, das 2-Nitro-1,3-indandion oder das 2-Acetyl-1,3-cyclo-hexandion.

Zu den erfindungsgemäß geeigneten Diketonen gehören auch cyclische Dicarbonylverbindungen wie z.B. das Isatin und dessen Derivate, wie sie z.B. in der deutschen Offenlegungsschrift DE 44 09 143 A1 beschrieben sind. Für das erfindungsgemäße Verfahren sind z.B. das Isatinsäure-Kaliumsalz, das Isatin-5-Sulfonsäure-Kaliumsalz, das N-Allylisatin, das 1-Piperidinomethylisatin, das 1-Hydroxymethylisatin und das 1-Diethylaminomethylisatin geeignet.

Eine weitere geeignete cyclische Dicarbonylverbindung ist z.B. auch die Dehydroascorbinsäure, deren Eignung als Haarfarbstoff aus der deutschen Patentanmeldung DE 19 745 354.6 bekannt ist. Schließlich eignen sich auch die Acetale, Iminderivate und Halbaminale der genannten reaktiven Carbonylverbindungen. Solche Verbindungen werden durch Reaktion der Carboxylgruppe mit primären Alkoholen oder Aminen und ggf. Wasserabspaltung erhalten.

Ausgehend von den ungesättigten Dialdehyden und Diketonen gelangt man dabei in die Gruppe der Merocyanin- und Azomethin-Farbstoffe. Geeignete Imin-Derivate des Glutacondialdehyds sind z.B. das Mono-N-methylanilin-Derivat des Glutaconaldehyds (5-N-Methylanilinopentadienal) oder das N-(5-Anilino-2,4-pentadien-1-yliden)-anilinium-chlorid. Ein weiterer geeigneter vinyloger Cyaninfarbstoff ist das 7-Dimethylamino-2,4,6-heptatrienyliden-dimethylammonium-perchlorat. Solche Verbindungen sind als Haarfärbe-mittel-Komponenten z.B. aus der deutschen Patentanmeldung DE 19 717 223.7 bekannt.

Viele der genannten reaktiven Carbonylverbindungen färben keratinhaltige Fasern unter Ausprägung verschiedenster Farbnuancen besonders intensiv erst in Kombination mit einer oder mehreren farbverstärkenden Verbindungen mit primärer oder sekundärer Amino- oder Hydroxygruppe, ausgewählt aus der Gruppe der Aminosäuren und Peptide, der aromatischen Amine, Phenole, Aminophenole und stickstoffhaltigen Heterocyclen.

Dabei werden in vielen Fällen auch tiefere (dunklere) Nuancen erhalten.

Geeignete Aminosäuren sind z.B. die natürlich vorkommenden und synthetischen Aminosäuren, z.B. Arginin, Histidin, Phenylalanin, Dihydroxyphenylalanin, Omithin, Lysin. Geeignete Peptide sind vor allem Oligo- und Polypeptide, die eine ausreichende Wasserlöslichkeit in den erfindungsgemäßen Zubereitungen zur Keratinreduktion aufweisen. Als Beispiele sind z.B. Glutathion oder die in den Hydrolysaten von Kollagen, Keratin, Elastin, Casein, Pflanzenproteinen wie Sojaprotein, Weizengluten oder Mandelprotein enthaltenen Oligopeptide zu nennen.

Geeignete aromatische Amine und Aminophenole sind N,N-Dimethyl-, N,N-Diethyl-, N-(2-Hydroxyethyl)-N-ethyl-, N,N-Bis-(2-hydroxyethyl)-, N-(2-Methoxyethyl-), 2-Chlor-, 2,3-, 2,4- und 2,5-Dichlor-p-phenylendiamin, 2,5-Dihydroxy-4-morpholinoanilindihydro-bromid, 2-, 3- und 4-Aminophenol, 2-Aminomethyl-4-aminophenol, 2-Hydroxymethyl-4-aminophenol, o- und p-Phenylendiamin, o- und m-Toluylendiamin, 2,5-Diamino-phenol,
-toluol und -phenethol, 4-Amino-3-methylphenol, 2-(2,5-Diaminophenyl)-ethanol, 2,4-Diaminophenoxy-ethanol, 2-(2,5-Diaminophenoxy)-ethanol, 4-Methylamino-, 3-Amino-4-(2'hydroxyethyloxy)-, 3,4-Methylendiamino- und 3,4-Methylendioxyanilin, 3-Amino-2,4-dichlor-, 4-Methylamino-, 2-Methyl-5-amino-, 3-Methyl-4-amino-, 2-Methyl-5-(2-hydro-xyethylamino)-, 6-Methyl-3-amino-2-chlor-, 2-Methyl-5-amino-4-chlor-, 3,4-Methylen-dioxy-, 5-(2-Hydroxyethylamino)-4-methoxy-2-methyl- und 4-Amino-2-hydroxymethyl-phenol, 1,3-Diamino-2,4-dimethoxybenzol, 2-, 3-, 4-Aminobenzoesäure, - phenylessig-säure, 2,3-, 2,4-, 2,5-, 3,4-, 3,5-Diaminobenzoesäure, 4-, 5-Aminosalicylsäure, 3-Amino-4-hydroxy-, 4-Amino-3-hydroxy-benzoesäure, 2-, 3-, 4-Aminobenzolsulfonsäure, 3-Amino-4-hydroxybenzolsulfonsäure, 4-Amino-3-hydroxynaphthalin-1-sulfonsäure, 6-Amino-7-hydroxynaphthalin-2-sulfonsäure, 7-Amino-4-hydroxynaphthalin-2-sulfonsäure, 4-Amino-5-hydroxynaphthalin-2,7-disulfonsäure, 3-Amino-2-naphthoesäure, 3-Aminophthalsäure, 5-Aminoisophthalsäure, 1,3,5-, 1,2,4-Triaminobenzol, 1,2,4,5-Tetraaminobenzol, 2,4,5-Triaminophenol, Pentaaminobenzol, Hexaaminobenzol, 2,4,6-Triaminoresorcin, 4,5-Diaminobrenzcatechin, 4,6-Diaminopyrogallol, 3,5-Diamino-4-hydroxybrenzcatechin, aromatische Aniline bzw. Phenole mit einem weiteren aromatischen Rest, wie sie in der Formel I dargestellt sind 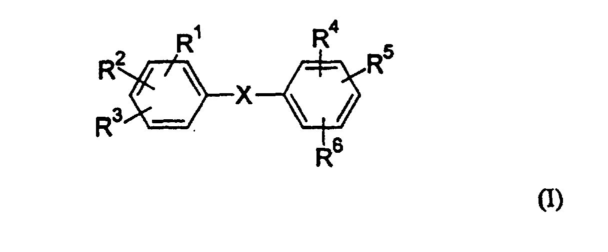 in der R¹ für eine Hydroxy- oder eine Aminogruppe, die durch C₁₋₄-Alkyl, C₁₋₄-Hydroxyalkyl- oder C₁₋₄-Alkoxy-C₁₋₄-alkyl substituiert sein kann, steht,
R², R³, R⁴, R⁵ und R⁶ für Wasserstoff, eine Hydroxy- oder eine Aminogruppe, die durch C₁₋₄-Alkyl-, C₁₋₄-Hydroxyalkyl, C₁₋₄-Aminoalkyl- oder C₁₋₄-Alkoxy-C₁₋₄-alkylgruppe substituiert sein kann, für eine Carbon- oder Sulfonsäuregruppe stehen, und
X für eine direkte Bindung, eine gesättigte oder ungesättigte, ggf. durch Hydroxygruppen substituierte Kohlenstoffkette mit 1 bis 4 Kohlenstoffatomen, eine Carbonyl-, Sulfonyloder Iminogruppe, ein Sauerstoff- oder Schwefelatom, oder eine Gruppe mit der Formel II

**Z-(CH**_{**2**}**-Y-CH**_{**2**}**-Z')**_{**o**} (II),

in der Y eine direkte Bindung, eine CH₂- oder CHOH-Gruppe bedeutet,
Z und Z' unabhängig voneinander ein Sauerstoffatom, eine NR⁷-Gruppe, worin R⁷ Wasserstoff, eine C₁₋₄-Alkyl- oder Hydroxy-C₁₋₄-alkylgruppe bedeutet, die Gruppe -O-(CH₂)ₚ-NH oder NH-(CH₂)_{p'}-O, worin p und p' 2 oder 3 sind, stehen und o eine Zahl von 1 bis 4 bedeutet,
wie beispielsweise 4,4'-Diaminostilben, 4,4'-Diaminostilben-2,2'-disulfonsäure-monooder -di-Na-Salz, 4,4'-Diaminodiphenylmethan, -sulfid, -sulfoxid, -amin, 4,4'-Diarninodiphenyl-amin-2-sulfonsäure, 4,4'-Diaminobenzophenon, -diphenylether, 3,3',4,4'-Tetraaminodi-phenyl, 3,3',4,4'-Tetraamino-benzophenon, 1,3-Bis-(2,4-diaminophenoxy)-propan, 1,8-Bis-(2,5-diaminophenoxy)-3,6-dioxaoctan, 1,3-Bis-(4-aminophenylamino)-propan, -2-propa-nol, 1,3-Bis-[N-(4-aminophenyl)-2-hydroxyethylamino]-2-propanol, N,N-Bis-[2-(4-amino-phenoxy)-ethyl]-methylamin, N-Phenyl-1,4-phenylendiamin.

Die vorgenannten Verbindungen können sowohl in freier Form als auch in Form ihrer physiologisch verträglichen Salze, insbesondere als Salze anorganischer Säuren, wie Salzoder Schwefelsäure, eingesetzt werden.

Geeignete Phenole sind z.B. das 2-, 3- oder 4-Methoxy-, das 3-Dimethylamino-, 2-(2-Hydroxyethyl)- und das 3,4-Methylendioxy-phenol, das Resorcin und das 2-, 4- und 5-Methylresorcin, das 2- und 4-Chloiresorcin, 2,5-Dimethylresorcin, Brenzkatechin, Hydrochinon, Pyrogallol, Phloroglucin, Hydroxyhydrochinon, die 2,4- oder 3,4-Dihydroxybenzoe- oder -phenylessigsäure, die Gallussäure, die 2,4,6-Trihydroxybenzoesäure oder das 2,4,5-Trihydroxyacetophenon, das 1-Naphthol, das 1,5-, 2,3- und 2,7-Dihydroxynaphthalin, die 6-Dimethylamino-4-hydroxy-2-naphthalinsulfonsäure oder die 3,6-Dihydroxy-2,7-naphthalindisulfonsäure.

Geeignete stickstoffhaltige heterocyclische Verbindungen sind z.B. 2-, 3-, 4-Amino-, 2-Amino-3-hydroxy-, 2,6-Diamino-, 2,5-Diamino-, 2,3-Diamino-, 2-Dimethylamino-5-amino-, 2-Methylamino-3-amino-6-methoxy-, 2,3-Diamino-6-methoxy-, 2,6-Dimethoxy-3,5-diamino-, 2,4,5-Triamino- und 2,6-Dihydroxy-3,4-dimethylpyridin, 2,4-Dihydroxy-5,6-diamino-, 4,5,6-Triamino-, 4-Hydroxy-2,5,6-triamino-, 2-Hydroxy-4,5,6-triamino-, 2,4,5,6-Tetraamino-, 2-Methylamino-4,5,6-triamino-, 2,4-, 4,5-Diamino- und 2-Amino-4-methoxy-6-methyl-pyrimidin, 3-Amino-, 3-Amino-5-hydroxy- und 3,5-Diaminapyrazol, 2-,3-, 8-Aminochinolin, 4-Amino-chinaldin, 2-, 6-Aminonicotinsäure, 5-Aminoisochinolin, 5-, 6-Aminoindazol, 5- und 7-Amino-benzimidazol und -benzothiazol, 2,5-Dihydroxy-4-morpholinoanilin sowie Indol- und Indolinderivate, wie 4-, 5-, 6- und 7-Aminoindol, 5,6-Dihydroxyindol, 5,6-Dihydroxyindolin und 4-Hydroxyindolin. Die vorgenannten Verbindungen können sowohl in freier Form als auch in Form ihrer physiologisch verträglichen Salze, z. B. als Salze anorganischer Säuren, wie Salz- oder Schwefelsäure, eingesetzt werden.

Diese Färbesysteme können noch weiter verstärkt werden durch geeignete stickstoffhaltige Heterocyclen wie z.B. Piperidin, Piperidin-2-, -3- oder -4-carbonsäure, Pyridin, 2-, 3- oder 4-Hydroxypyridin, Imidazol, 1-Methylimidazol, Histidin, Pyrrolidin, Pyrrolidon, Pyrrolidon-5-carbonsäure, Pyrazol, 1,2,4-Triazol, Piperazin sowie deren physiologisch verträglichen Salze.

In der keratinreduzierenden Zubereitung für das erfindungsgemäße Verfahren können mehrere Farbstoffvorprodukte vom Typ der reaktiven Carbonylverbindungen gleichzeitig enthalten sein. Desgleichen können auch mehrere Verbindungen aus der Gruppe der Aminosäuren, Peptide, aromatischen Amine, Aminophenole, Phenole und stickstoffhaltigen Heterocyclen gemeinsam enthalten sein, wenn dies zur Erzielung der gewünschten Farbnuance erforderlich ist.

Viele der als farbgebende Kombinationskomponenten eingesetzten Verbindungen aus der Gruppe der aromatischen Amine, Phenole, Aminophenole oder stickstoffhaltigen Heterocyclen sind klassische Oxidationsfarbstoffvorprodukte, die auch ohne die aktivierten Carbonylverbindungen unter dem Einfluß des Oxidationsmittels der Fixierstufe eine Haarfärbung ausbilden. Durch geeignete Kombination mit der aktivierten Carbonylverbindung läßt sich der Farbton im Fixierprozeß noch weiter variieren. Man erhält auf diese Weise eine breite Palette von Nuancen, die sich durch Brillanz, hohe Echtheit und gute Stabilität gegenüber dem Waschen der Haare auszeichnen.
Im übrigen kann die keratinreduzierende Zubereitung, wie für Dauerwellverfahren üblich, zusammengesetzt sein. Sie enthält daher eine keratinreduzierende Substanz in einer zur wirksamen Reduzierung der Cystin-Disulfidbrücken des Haarkeratins unter physiologisch erträglichen Bedingungen, d.h. bei Temperaturen unter 40°C und in einer Einwirkungszeit von maximal 40 Minuten erforderlichen Menge. Als keratinreduzierende Stoffe werden bevorzugt Mercaptoverbindungen oder Sulfite (Alkalisalze der schwefligen Säure) eingesetzt. Geeignete Mercaptoverbindungen sind z.B. Thioglycolsäure, Thioglycolsäureglycerinester, Thiomilchsäure, Cystein und Cysteamin, Thioglycerin, Mercaptoethanol oder N-(2-Mercapioethyl)-N,N-dimethylammonioacetat.

Ein weiterer Erfindungsgegenstand ist daher die Zubereitung zur Durchführung der keratinreduzierenden Stufe in einem Verfahren zur gleichzeitigen dauerhaften Verformung und Färbung der Haare, die eine keratinreduzierende Substanz, ausgewählt aus der Gruppe der Mercaptoverbindungen und der Sulfite und einen Farbstoff vom Typ der reaktiven Carbonylverbindungen, ausgewählt aus der Gruppe der aromatischen oder ungesättigten Aldehyde oder Ketone, der Dialdehyde oder Diketone oder deren Acetale oder Iminderivate in einem wäßrigen oder wäßrig-alkoholischen Träger enthält. Zur Intensivierung und farblichen Variation der Nuancen ist vorzugsweise zusätzlich wenigstens eine Verbindung aus der Gruppe der aliphatischen oder aromatischen Amine, der Phenole, der Aminophenole oder der stickstoffhaltigen Heterocyclen enthalten.

Als keratinreduzierende Substanz ist bevorzugt ein Salz oder ein Ester der Thioglycolsäure oder der Thiomilchsäure in einer Menge von 2 - 20 Gew.-% enthalten. Die aktivierte Carbonylverbindung ist bevorzugt in einer Menge von 0,5 - 5 Gew.-% enthalten.

Von den zur Farbstellung des Systems sowie zur Intensivierung und farblichen Variation der Nuancen erforderlichen Verbindungen sind besonders bevorzugt die aromatischen Amine und Aminophenole in einer Menge von 0,01 - 5 Gew.-% enthalten. Darüber hinaus kann die erfindungsgemäße Zubereitung zur Durchführung der keratinreduzierenden Stufe des erfindungsgemäßen Farbdauerwell-Verfahrens im wäßrigen oder wäßrig-alkoholischen Träger alle hierfür bekannten Hilfsmittel und Zusatzstoffe enthalten. Dies sind in erster Linie oberflächenaktive Stoffe und Puffersalze. Darüber hinaus können auch Ammoniak zur Einstellung eines pH-Wertes von 6,5 - 10, Harnstoff, Glycole, Glycerin, Polyethylenglycol, wasserlösliche Lösungsmittel, wasserlösliche Polymere zur Viskositäts-erhöhung, Duftstoffe oder Trübungsmittel zugesetzt werden. Eine besondere Rolle kann dabei Hilfsmitteln mit einer die keratinreduzierenden Substanzen verstärkenden Wirkung zukommen. Solche Hilfsmittel sind z.B. Propylenglycolmonomethylether und bestimmte Diole wie 1,3-Butandiol, 1,2-Pentandiol, 1,6-Hexandiol oder 2-Ethylhexandiol-1,3. Auch bestimmte heterocyclische Verbindungen wie z.B. Imidazol, Pyrrolidin, Piperidin, Dioxan, Dioxolan, Morpholin und Piperazin verstärken die Wirkung der keratinreduzierenden Mittel. Bevorzugt sind als wellverstärkende Zusätze Imidazol oder 2-Ethylhexandiol-1,3 in einer Menge von 0,1 - 5 Gew.-% in den erfindungsgemäßen Zubereitungen enthalten.

Als oberflächenaktive Stoffe eignen sich alle wasserlöslichen Tenside in Mengen von 0,1 - 10 Gew.-%. Die Tenside dienen in erster Linie der Emulgierung oder Solubilisierung der Duftstoffe, die zur Überdeckung des Eigengeruches der Mercaptoverbindungen enthalten sind. Man verwendet daher bevorzugt nichtionische Tenside mit gutem Parfümöl-Solubilisierungsvermögen. Solche Tenside sind z.B. die oxethylierten Fettalkohole, oxethylierten Fettsäuren, oxethylierte Fettsäurepartialglyceride, oxethylierte Sorbitan-fettsäureester, oxethyliertes und ggf. hydriertes Rizinusöl, Alkyl-(oligo)-glucoside und oxethylierte Methylglucosid-fettsäureester.

Als Puffersalze eigenen sich bevorzugt Ammoniumsalze schwacher Säuren, die in den Zubereitungen einen pH-Bereich von pH = 6,5 bis 10 stabilisieren können. Geeignete Puffersalze sind z.B. (NH₄)₂CO₃, Ammoniumborat, Ammoniumphosphat, Triethanolammoniumcitrat oder Ammoniumglycinat.

Die erfindungsgemäße Zubereitung zur Durchführung der keratinreduzierenden Stufe des erfindungsgemäßen Farbdauerwell-Verfahrens kann auch als wäßriges Schaumaerosol konfektioniert sein, das mit einem verflüssigten Gas, z.B. mit Propan-Butan-Gemischen, N₂O (Stickoxydul), Dimethylether, Fluorkohlenwasserstoffen (z.B. HFC 134a) oder Gemischen davon in Aerosolbehältern mit Schaumventil abgefüllt ist.

Die erfindungsgemäße Zubereitung kann vor oder nach dem Aufwickeln des Haars auf die mechanische Verformungshilfe, z.B. auf Wickler oder Papilloten, auf das Haar aufgebracht werden. Zur Reduktion des Haarkeratins und zum Eindringen der Farbstoffe in das Haar sollte eine Verweilzeit von wenigstens 5 - 10 Minuten, bevorzugt von 10 - 30 Minuten, jedoch möglichst nicht länger als 40 Minuten vorgesehen sein. Danach sollte die Zubereitung mit Wasser, ggf. unter Zusatz von oberflächenaktiven Stoffen gespült und mit der Zubereitung eines Oxidationsmittels fixiert werden.

Als Fixierlösung eignet sich bevorzugt eine wäßrige Zubereitung, die als Oxidationsmittel Wasserstoffperoxid oder Kaliumbromat, bevorzugt in einer Menge von 0,5 - 10 Gew.-% enthält und einen pH-Wert von 2 - 6, bevorzugt von 2 - 4, aufweist. Zur Stabilisierung des Wasserstoffperoxids kann ein üblicher Peroxidstabilisator, z.B. ein Komplexbildner, enthalten sein. Darüber hinaus können oberflächenaktive Stoffe, z.B. kationische wasserlösliche Tenside oder Betaintenside, kationische wasserlösliche Polymere, Vitamine, Proteine oder Proteinhydrolysate und andere haarkosmetische Wirkstoffe enthalten sein.

Die Zubereitung des Oxidationsmittels kann auch als Feststoff formuliert sein, der als Oxidationsmittel z.B. Kalium- oder Natriumbromat oder ein festes Perhydrat (z.B. Melamin-perhydrat oder Hamstoffperhydrat) enthält und der erst kurz vor der Anwendung in dem erfindungsgemäßen Verfahren in Wasser aufgelöst wird. Die Oxidationsmittel-Zubereitung wird auf das Haar gebracht und dort ebenfalls eine gewisse Zeit, z.B. 1 - 5 Minuten einwirken gelassen. Dann wird das Haar mit Wasser, das ggf. ein gelöstes Salz eines 2- oder 3-wertigen Metalls, z.B. ein Magnesium-, Calcium- oder Aluminiumsalz, enthalten kann, gespült.

Schließlich wird das Haar getrocknet und von den mechanischen Verformungshilfen (Wickler, Papilloten) befreit.

Die folgenden Beispiele sollen das erfindungsgemäße Verfahren näher erläutern:

### Beispiele

Keratinreduzierende und gleichzeitig färbende Zubereitungen der Zusammensetzungen 1 - 5 der Tabelle 1 wurden hergestellt und in einem Dauerwellverfahren eingesetzt.

Aufgewickelte Haarsträhnen aus blondem Humanhaar wurden mit den Zubereitungen 1-5 30 Minuten bei 20°C benetzt. Anschließend wurden sie mit Wasser gespült und weitere 30 Minuten (bei 20°C) mit einer 3 %igen Lösung von Wasserstoffperoxid behandelt (fixiert). Danach wurde erneut mit Wasser gespült und getrocknet. Dann wurden die Wickler entfernt.

Die erhaltenen Farbresultate sind aus der Tabelle 1 zu entnehmen.

**Tabelle 1**

| | **1** | **2** | **3** | **4** | **5** |
|---|---|---|---|---|---|
| Keratinreduktionsmittel: | | | | | |
| Thioglycolsäure | 8,0 | 8,0 | 8,0 | 8.0 | 8,0 |

| Puffersalz: | | | | | |
|---|---|---|---|---|---|
| Ammoniumcarbonat | 3,0 | 3,0 | 3,0 | 3,0 | 3,0 |

| aktivierte Carbonylverbindung: | | | | | |
|---|---|---|---|---|---|
| Glutaconaldehyd, Na-Salz | 0,8 | 0,8 | 1,6 | - | - |
| 1-Diethylaminomethylisatin | - | - | - | 1,2 | 1,2 |

| Farbverstärker-Verbindung: | | | | | |
|---|---|---|---|---|---|
| 2-Aminomethyl-4-aminophenol | 1,0 | 1,0 | 2,0 | - | - |
| 2-Methylamino-3-amino- | | | | | |
| 6-methoxypyridin ( 2HCl) | - | - | - | 1,1 | - |
| 1,8-Bis-(2,5-diaminophenoxy)- | | | | | |
| 3,6-dioxaoctan ( 4HCl) | - | - | - | - | 2,5 |
| 5,6-Dihydroxyindolin ( HBr) | - | 0,1 | - | - | - |

| Träger: | | | | | |
|---|---|---|---|---|---|
| Cremophor RH60 | 3,0 | 3,0 | 3,0 | 3,0 | 3,0 |
| NH₃ | pH = | pH = | pH = | pH = | pH = |
| Wasser | ad 100 | ad 100 | ad 100 | ad 100 | ad 100 |
| erhaltene Nuance | gold-blond | natur-blond | hell-kupfer | reh-braun | mittel-braun |

## Patentansprüche

1. Verfahren zur gleichzeitigen dauerhaften Verformung und Färbung der Haare. bei dem die Haare vor und/oder nach einer mechanischen Verformung mit einem Keratinreduktionsmittel behandelt, gespült und mit einem Oxidationsmittel fixiert werden, **dadurch gekennzeichnet, daß** man zur Keratinreduktion eine wäßrige Zubereitung verwendet, die eine Mercaptoverbindung oder ein Sulfit und wenigstens einen Farbstoff vom Typ der reaktiven Carbonylverbindungen, ausgewählt aus der Gruppe der aromatischen, heteroaromatischen oder ungesättigten Aldehyde oder Ketone, der Dialdehyde oder Diketone oder deren Acetale, Halbaminale oder Iminderivate enthält.

2. Verfahren nach Anspruch 1, **dadurch gekennzeichnet, daß** man zur Keratinreduktion eine wäßrige Zubereitung verwendet, die zusätzlich wenigstens eine Verbindung aus der Gruppe der Aminosäuren und Peptide, der aromatischen Amine. Phenole, Aminophenole oder der stickstoffhaltigen Heterocyclen enthält.

3. Zubereitung zur Durchführung der keratinreduzierenden Stufe in einem Verfahren zur gleichzeitigen dauerhaften Verformung und Färbung der Haare, enthaltend eine keratinreduzierende Substanz, ausgewählt aus der Gruppe der Mercaptoverbindungen und der Sulfite und einem Farbstoff vom Typ der reaktiven Carbonylverbindungen, ausgewählt aus der Gruppe der aromatischen oder ungesättigten Aldehyde oder Ketone, der Dialdehyde oder Diketone oder deren Acetale, Halbaminale oder Iminderivate in einem wäßrigen oder wäßrig-alkoholischen Träger.

4. Zubereitung nach Anspruch 3, **dadurch gekennzeichnet, daß** zusätzlich eine Verbindung aus der Gruppe der aliphatischen oder aromatischen Amine, der Phenole, Amino-phenole oder der stickstoffhaltigen Heterocyclen enthalten ist.

5. Zubereitung nach Anspruch 3 oder 4, **dadurch gekennzeichnet, daß** als keratinreduzierende Substanz ein Salz oder ein Ester der Thioglycolsäure oder der Thiomilchsäure in einer Menge von 2 - 20 Gew.-% und eine aktivierte Carbonylverbindung in einer Menge von 0,5 - 5 Gew.-% enthalten ist.

6. Zubereitung nach einem der Ansprüche 3 - 5, **dadurch gekennzeichnet, daß** ein aromatisches Amin oder Aminophenol in einer Menge von 0,01 - 5 Gew.-% enthalten ist.

7. Zubereitung nach einem der Ansprüche 3 - 6, **dadurch gekennzeichnet, daß** der wäßrige oder wäßrig-alkoholische Träger wenigstens eine oberflächenaktive Verbindung und Puffersalze enthält.

8. Zubereitung nach dem der Ansprüche 3 - 7, **dadurch gekennzeichnet, daß** der wäßrige oder wäßrig-alkoholische Träger wenigstens einen wellverstärkenden Zusatz wie Imidazol oder 2-Ethyl-hexandiol-1,3 enthält.

## Claims

1. A process for the simultaneous permanent shaping and colouring of hair, in which the hair is treated with a keratin reducing agent before and/or after mechanical deformation, rinsed and fixed with an oxidizing agent, **characterized in that** the keratin reducing step is carried out with an aqueous preparation containing a mercapto compound or a sulfite and at least one dye of the reactive carbonyl compound type selected from the group of aromatic, heteroaromatic or unsaturated aldehydes or ketones, dialdehydes or diketones or their acetals, semiaminals or imine derivatives.

2. A process as claimed in claim 1, **characterized in that** the keratin reducing step is carried out with an aqueous preparation additionally containing at least one compound from the group of amino acids and peptides, aromatic amines, phenols, aminophenols or nitrogen-containing heterocycles.

3. A preparation for carrying out the reducing step of a process for the simultaneous permanent shaping and colouring of hair containing a keratin-reducing substance selected from the group of mercapto compounds and sulfites and a dye of the reactive carbonyl compound type selected from the group of aromatic or unsaturated aldehydes or ketones, dialdehydes or diketones or their acetals, semiaminals or imine derivatives in an aqueous or aqueous/alcoholic carrier.

4. A preparation as claimed in claim 3, **characterized in that** it additionally contains a compound from the group of aliphatic or aromatic amines, phenols, aminophenols or nitrogen-containing heterocycles.

5. A preparation as claimed in claim 3 or 4, **characterized in that** the keratin reducing substance present is a salt or an ester of thioglycolic acid or thiolactic acid in a quantity of 2 to 20% by weight and an activated carbonyl compound in a quantity of 0.5 to 5% by weight.

6. A preparation as claimed in any of claims 3 to 5, **characterized in that** an aromatic amine or aminophenol is present in a quantity of 0.01 to 5% by weight.

7. A preparation as claimed in any of claims 3 to 6, **characterized in that** the aqueous or aqueous/alcoholic carrier contains at least one surface-active compound and buffer salts.

8. A preparation as claimed in claims 3 to 7, **characterized in that** the aqueous or aqueous/alcoholic carrier contains at least one wave-strengthening additive, such as imidazole or 2-ethylhexane-1,3-diol.

## Revendications

1. Procédé de mise en plis et de coloration durables simultanées des cheveux, dans lequel on traite les cheveux avant et/ou après une mise en plis mécanique, avec un réducteur de kératine, on rince et on fixe avec un oxydant, **caractérisé en ce que** pour la réduction de la kératine on utilise une préparation aqueuse qui contient un composé mercapto ou un sulfite et au moins un colorant du type des composés carbonyle réactifs, choisi dans le groupe des aldéhydes ou des cétones aromatiques, hétéroaromatiques ou insaturés, des dialdéhydes ou des dicétones ou de leurs acétals, hémi-aminals ou dérivés imine.

2. Procédé selon la revendication 1, **caractérisé en ce que** pour la réduction de la kératine on utilise une préparation aqueuse qui contient en outre au moins un composé du groupe des acides aminés et des peptides, des amines aromatiques, des phénols, des aminophénols ou des hétérocycles contenant de l'azote.

3. Préparation pour réaliser l'étape de réduction de la kératine dans un procédé de mise en plis et de coloration durables simultanées des cheveux, contenant une substance réduisant la kératine, choisie dans le groupe des composés mercapto et des sulfites et un colorant du type des composés carbonyle réactifs, choisi dans le groupe des aldéhydes ou cétones aromatiques ou insaturés, des dialdéhydes ou des dicétones ou de leurs acétals, hémi-aminals ou dérivés imine dans un support aqueux ou alcoolique-aqueux.

4. Préparation selon la revendication 3, **caractérisée en ce qu'**un composé du groupe des amines aliphatiques ou aromatiques, des phénols, des aminophénols ou des hétérocycles contenant de l'azote y est contenu.

5. Préparation selon la revendication 3 ou 4, **caractérisée en ce que** comme substance réduisant la kératine, un sel ou un ester de l'acide thioglycolique ou de l'acide thiolactique en une quantité de 2 à 20% en poids et un composé carbonyle activé en une quantité de 0,5 à 5% en poids y est contenu.

6. Préparation selon l'une des revendications 3 à 5, **caractérisée en ce qu'**une amine aromatique ou un aminophénol en une quantité de 0,01 à 5% en poids y est contenue.

7. Préparation selon l'une des revendications 3 à 6, **caractérisée en ce que** le support aqueux ou alcoolique aqueux contient au moins un composé tensioactif et des sels tampon.

8. Préparation selon les revendications 3 à 7, **caractérisée en ce que** le support aqueux ou alcoolique aqueux contient au moins un additif renforçant l'ondulation comme l'imidazole ou le 2-éthyl-hexanediol-1,3.
